# EUROPÄISCHE PATENTSCHRIFT

(11) **EP 0 743 835 B1**
(45) Veröffentlichungstag und Bekanntmachung des Hinweises auf die Patenterteilung: **06.05.1998**
(21) Anmeldenummer: 95909740.3
(22) Anmeldetag: 18.02.1995
(51) Int. Cl.: A61B 17/04

(54) **MEDIZINISCHES BESTECK ZUM PLAZIEREN UND VERSCHIEBEN VON KNOTEN BEI OPERATIVEN EINGRIFFEN**
MEDICAL INSTRUMENT FOR PLACING AND DISPLACING KNOTS DURING SURGICAL OPERATIONS
INSTRUMENT MEDICAL POUR PLACER ET DEPLACER DES NOEUDS LORS D'INTERVENTIONS CHIRURGICALES

(30) Priorität: 25.02.1994 DE 4406203
(43) Veröffentlichungstag der Anmeldung: 27.11.1996
(73) Patentinhaber: Fleega, Basim A., 53117 Bonn (DE)
(72) Erfinder: Fleega, Basim A., 53117 Bonn (DE)
(74) Vertreter: Müller-Gerbes, Margot, Dipl.-Ing.
(86) Internationale Anmeldenummer: EP9500592
(87) Internationale Veröffentlichungsnummer: WO9522931

(56) Entgegenhaltungen:
- DE-U- 9 100 600
- DE-U- 9 112 301
- DE-U- 9 112 532
- DE-U-29 502 778

## Beschreibung

Die Erfindung betrifft ein medizinisches Besteck zum Verschieben und Plazieren von mittels chirurgischer Fäden hergestellter Knoten bei operativen Eingriffen mit einem in einer zylindrischen Hülse längsverschieblichen zylindrischen Stab, der an seinen beiden Enden über die Hülse vorsteht, wobei der Stab ein in die Hülse hineinziehbares Kopfende und ein am anderen Ende der Hülse außenseitig anschlagbares Griffende aufweist und von dem stirnseitigen Kopfende sich zwei durchgehende Bohrungen zum Durchstecken eines Fadens zur zylindrischen Mantelfläche des Stabes erstrecken und das Hülsenende, in das der Stab einziehbar ist, als Knotencutter mit Schneidkante ausgebildet ist, um die Fadenenden vom Knoten abzutrennen.

Aus der DE-U-9 112 532 ist ein Fadenabschneider der gattungsgemäßen Art bekannt, mit dem es möglich ist, ein oder mehr Fäden bei operativen Eingriffen auch an schwer zugänglichen Stellen abzuschneiden.

Solch ein operativer Eingriff, bei dem Fäden gelegt und verknotet werden und an bestimmte Stellen gebracht werden müssen, findet beispielsweise bei der Rekonstruktion eines Rotatoren-Manschettenrisses statt, bei dem Sehnen genäht werden müssen.

Bei einer konventionellen Rekonstruktion eines Rotatoren-Manschettenrisses war es bis jetzt stets nötig, die betroffene Schulterpartie des Patienten relativ großflächig zu öffnen, um ausreichend Operationsraum zu schaffen. Derartig großflächige Eingriffe sind aber stets mit sehr hohen Belastungen für den Patienten verbunden und erfordern eine entsprechend lange Rekonvaleszenz.

Man ist daher bemüht, operative Eingriffe als transarthroskopische Eingriffe mit entsprechend kleinen Operationsöffnungen durchzuführen, so daß die Nachteile der konventionellen Operationsmethoden vermieden werden. Für die Durchführung solcher operativen transarthroskopischen Eingriffe und Rekonstruktionen auch mit der Folge des Nähens von Rissen erfordern jedoch entsprechend angepaßte und neue Bestecke, um auch bei entsprechend kleinen Operationsöffnungen die notwendigen Arbeiten durchführen zu können.

Der vorliegenden Erfindung liegt die Aufgabe zugrunde, ein Besteck zu schaffen, mit dem es möglich ist, eine operative transarthroskopische Rekonstruktion, beispielsweise eines Rotatoren-Manschettenrisses durchzuführen, bei dem Fäden gelegt, verknotet, festgezogen und an den geeigneten Ort gebracht werden.

Zur Lösung der gestellten Aufgabe schlägt die Erfindung die Weiterbildung eines gattungsgemäßen medizinischen Bestecks zum Plazieren und Verschieben von mittels chirurgischer Fäden hergestellter Knoten bei operativen Eingriffen vor, das sich dadurch auszeichnet, daß eine weitere Durchgangsbohrung zum Durchziehen eines Fadens im Bereich des Kopfendes des Stabes ausgebildet ist, die quer zur Längsachse des zylindrischen Stabes angeordnet ist, wodurch das Kopfende des zylindrischen Stabes als Knotenschieber zum Verschieben und Festziehen eines mittels der Fäden hergestellten Knotens ausgebildet ist.

Mit dem erfindungsgemäß vorgeschlagenen Knotenschieber und Knotencutter ist eine einwandfreie Verknotung und damit Fixierung der mittels Operationsfäden wieder zusammengeführten Teile, wie Sehnen, auf ihre ursprünglichen Befestigungsflächen zu erreichen.

Vorteilhafte Ausgestaltungen des erfindungsgemäßen Besteckes, das als Knotenschieber und Knotencutter dient, sind den kennzeichnenden Merkmalen der Unteransprüche entnehmbar. Insbesondere ist der zylindrische Stab als Knotenschieber mit einem konvex abgerundeten Kopfende ausgebildet und von dem stirnseitigen Kopfende erstrecken sich zwei durchgehende Bohrungen zum Durchstecken eines Fadens zur zylindrischen Mantelfläche des Stabes. Diese beiden durchgehenden Bohrungen dienen sozusagen als Nadelöhr, um jeweils ein Fadenende hindurchzuführen. Bevorzugt sind diese beiden durchgehenden als Nadelöhre benutzte Bohrungen diametral einander gegenüberliegend auf dem Kopfende des Stabes ausgebildet, wobei die Bohrungen sich von der Stirnseite des Stabes schräg zur Mantelfläche hin erstrecken. Diese beiden durchgehenden als Nadelöhre benutzten Bohrungen sind relativ klein und kurz, insbesondere 2 bis 4 mm lang bei einem Durchmesser von 1 bis 2 mm. Des weiteren ist der erfindungsgemäße Knotenschieber und Knotencutter mit einer weiteren Durchgangsbohrung zum Durchziehen eines Fadens im Bereich des Kopfendes des zylindrischen Stabes ausgebildet, wobei diese Bohrung quer zur Längsachse des zylindrischen Stabes angeordnet ist, und zwar so, daß sie die Bohrungen, die als Nadelöhr dienen, nicht tangieren. Die querverlaufende Durchgangsbohrung ist bevorzugt etwas unterhalb der Austritte der beiden vom stirnseitigen Kopfende ausgehenden Bohrungen an der zylindrischen Mantelfläche des Stabes kurz unterhalb dieser angeordnet. Um beim Durchziehen eines Fadens durch die querverlaufende Bohrung ein Scheuern desselben zu vermeiden, ist vorgesehen, daß die querverlaufende Bohrung im Eingangsbereich bzw. Austrittsbereich an der zylindrischen Mantelfläche des Stabes zumindest in dem dem Kopfende des Stabes abgewandten Bereichen abgerundet ist. Auch ist vorgesehen, daß die querverlaufende Bohrung einen etwas größeren Durchmesser als die beiden als Nadelöhr dienenden schräg verlaufenden Bohrungen, die vom stirnseitigen Kopfende ausgehen, aufweist. Hierbei kann der Durchmesser der querverlaufenden Bohrung zwischen 3 und 4 mm betragen.

Ein Operationsbesteck, um beispielsweise eine operative transarthroskopische Rekonstruktion eines Rotatoren-Manschettenrisses durchzuführen, umfaßt im wesentlichen eine Knochennadel, einen Sehnennadel, eine Arthroskopie-Gerade-Schere, den erfindungsgemäßen Knotenschieber und Knotencutter, einen Fadenleger und Fadenfänger, eine Sehnenraspel und einen Knochennadelhalter.

Um die Rekonstruktion des Rotatoren-Manschettenrisses durchzuführen, muß die ab- bzw. eingerissene Sehne mittels entsprechender Operationsfäden wieder an ihrem ursprünglichen Platz fixiert werden. Zu diesem Zweck wird ein Fadenleger und -fänger vorgeschlagen, der aus zwei gegeneinander verschiebbar gelagerten Elementen zusammensetzbar ist, von denen das eine Element als Fadenleger und das andere als Fadenfänger ausgebildet ist. Der Fadenleger ist dabei in Gestalt einer geraden, am Ende hakenförmig abgebogenen durchgängigen Hohlnadel zum Durchführen eines Fadens ausgeführt und weist einen Führungszylinder im geraden Teil der Nadel fest angebracht auf, der eine durchgehende Bohrung zum Durchführen des Fadenfängers aufweist. Die Durchführungsbohrung ermöglicht eine im wesentlichen parallele Führung des Fadenfängers zum geraden Teil des Fadenlegers.

Der Fadenfänger enthält einen äußeren Hohlzylinder zum Durchführen durch die Durchführungsöffnung des Fadenlegers und einen inneren Zylinder, der im Hohlzylinder längsverschiebbar ist. Eine Drahtschlaufe, die am inneren Zylinder befestigt ist, ist durch Öffnungen im äußeren Hohlzylinder nach außen geführt und kann durch Bewegung des inneren Zylinders längs des Hohlzylinders an die Außenfläche des Hohlzylinders angelegt bzw. von diesem beabstandet werden.

Für eine einwandfreie Verknotung und somit Fixierung der mittels Operationsfäden wieder zusammengeführten Sehnenbereiche auf ihre ursprünglichen Befestigungsflächen zu erreichen, wird der erfindungsgemäße Knotenschieber und -cutter benutzt.

Mit Hilfe des Knotenschiebers und -cutters des erfindungsgemäßen Besteckes ist es möglich, die beiden Enden eines Operationsfadens außerhalb der Operationsöffnung miteinander zu verknoten und die Knotenenden durch die im zylindrischen Stab angeordneten Durchgangsbohrungen hindurchzuführen. Auf diese Weise kann der nun vor der konvexen Stirnseite des zylindrischen Stabes liegende Knoten durch die Operationsöffnung hindurch bis zum gewünschten Verknotungsbereich vor dem zylindrischen Stab hergeschoben werden. Mittels Durchführen lediglich eines Fadenendes durch eine Durchgangsbohrung, vorzugsweise durch die zusätzliche Durchgangsbohrung zu den zwei durchgehenden Bohrungen, die sich zur konvexen Stirnseite des zylindrischen Stabes erstrecken, kann darüber hinaus ein derartig vorgeschobener Knoten unter gleichzeitigem Ziehen an beiden Fadenenden verfestigt werden.

Um nun einen derartig gelegten Knoten an seinen Enden dicht an der Verknotungsstelle von dem übrigen Faden abzutrennen, sieht die Erfindung vor, an der längsverschiebbaren zylindrischen Hülse auf dem zylindrischen Stab des zum erfindungsgemäßen Besteck gehörenden Knotenschiebers und -cutters an dem dem stirnseitigen Ende des zylindrischen Stabes zugewandten Ende der zylindrischen Hülse einen scharfen Anschliff - Schneidkante - zum Durchtrennen des aus den Bohrungen am Umfang des zylindrischen Stabes austretenden Fadens anzuordnen.

Ein komplettes Besteck zum Durchführen einer operativen transarthroskopischen Rekonstruktion eines Rotatoren-Manschettenrisses und ihre Anwendung werden nachfolgend in den Figuren näher erläutert. Es zeigen
- Fig. 1: einen Schnitt durch eine Arthroskopie-Gerade-Schere
- Fig. 2a: eine Seitenansicht einer Knochennadel
- Fig. 2b: einen Schnitt gemäß den Pfeilen AA in Fig. 2a
- Fig. 3a: eine Seitenansicht einer Sehnennadel
- Fig. 3b: einen Schnitt gemäß den Pfeilen BB in Fig. 3a
- Fig. 4a: einen Fadenleger in der Seitenansicht
- Fig. 4b: einen Fadenfänger in der Seitenansicht
- Fig. 4c: einen Schnitt durch die Einzelheit X in Fig. 4b
- Fig. 4d: eine weitere Ansicht der Einzelheit X gemäß Schnitt X1-X1 von Fig. 4c
- Fig. 4e: einen Fadenleger und -fänger, der aus einem Fadenleger gemäß Fig. 4a und einem Fadenfänger gemäß Fig. 4b zusammengesteckt ist
- Fig. 4f: eine vergrößerte Darstellung des Endbereiches des Fadenfängers mit eingefangenem Faden
- Fig. 5a: eine teilweise geschnittene Ansicht einer Hülse für einen Knotenschieber und Knotencutter
- Fig. 5b: eine Ansicht des zylindrischen Stabes für einen Knotenschieber und Knotencutter
- Fig. 5c: eine teilweise geschnittene Ansicht des aus Hülse und Stab gemäß Fig. 5a und 5b zusammengesetzten Knotenschiebers und Knotencutters
- Fig. 5d: die Einzelheit Y aus Fig. 5b in vergrößerter Darstellung
- Fig. 5e: einen Längsschnitt durch die Einzelheit Y gemäß Fig. 5d
- Fig. 5f: die Aufsicht auf das Kopfende der Einzelheit Y gemäß Fig. 5d
- Fig. 5g: einen um 90° versetzten Längsschnitt durch die Einzelheit Y gemäß fig. 5d
- Fig. 5h: eine weitere Variante eines Knotenschiebers und Knotencutters gemäß Fig. 5c
- Fig. 5i: die Einzelheit Z gemäß Fig. 5h aus einer um 90° gedrehten Position
- Fig. 5k: eine Aufsicht auf das Kopfende der Einzelheit Z gemäß Fig. 5i.
- Fig. 6a-c: in schematischer Darstellung den Einsatz des Knotenschiebers und -cutters
- Fig. 7a-c: in schematischer Darstellung den Einsatz des Fadenlegers und -fängers
- Fig. 8a: eine unverletzte Schulterpartie
- Fig. 8b: einen Rotatoren-Manschettenriß
- Fig. 8c-h: in schematischer Darstellung den Einsatz des erfindungsgemäßen Besteckes bei einer operativen transarthroskopischen Rekonstruktion eines Rotatoren-Manschettenrisses gemäß Fig. 8b zum Herstellen einer transosären Naht
- Fig. 9a,b: den Einsatz des erfindungsgemäßen Besteckes bei einer weiteren operativen transarthroskopischen Rekonstruktion eines anderen Rotatoren-Manschettenrisses mit einer End-zu-End-Sehnennaht.

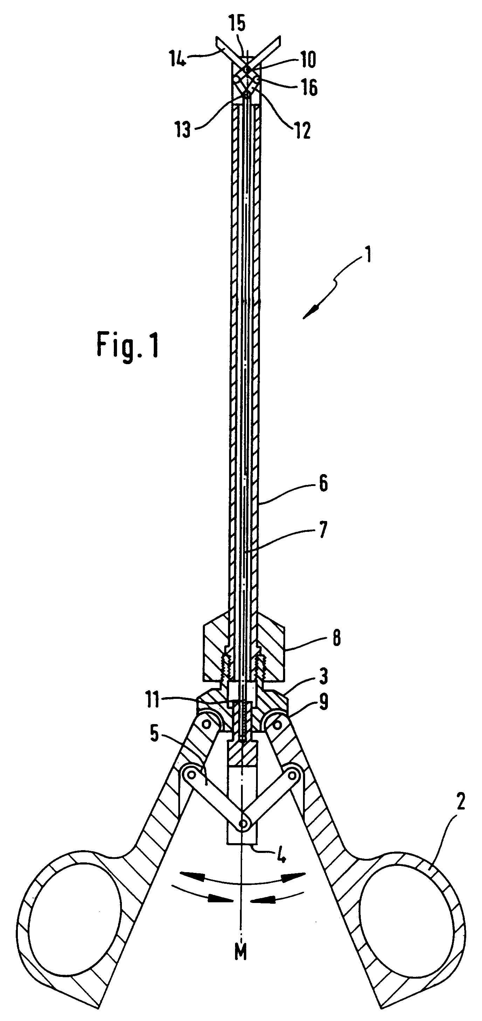

Gemäß der Fig. 1 besteht die Arthroskopie-Gerade-Schere 1 des erfindungsgemäßen Besteckes aus zwei Schenkeln mit ösenförmigen Handgriffen 2, die im Scherenlager 3 am Bolzen 9 schwenkbar angelenkt sind. In dem Scherenlager 3 ist eine Verlängerung 6 in Gestalt eines zylindrischen Hohlstabes befestigt, beispielsweise eingeschraubt oder eingelegt und mittels einer Überwurfmutter 8 am Scherenlager 3 befestigt. Die Länge der Verlängerung 6 wird durch die erforderliche Operationstiefe bestimmt. Am anderen Ende der Verlängerung sind die Schneiden 14 um eine gemeinsame Mittelachse 10 drehbar gelagert. Die Verlängerung 6 weist dazu an ihrem Ende eine Ausnehmung 15 auf, um die Bewegung der Schneiden 14 zu gestatten. Die Betätigung der Schneiden erfolgt nun über die Handgriffe 2 dergestalt, daß Gelenkstäbe 5 an ihrer einen Seite an den Handgriffen 2 drehbar angebracht sind und an ihrer anderen Seite an einem Gleitstein 4 befestigt sind, der im Scherenlager 3 verschiebbar ist. Um eine gleichmäßige Betätigung des Gleitsteines 4 zu ermöglichen, ist dieser dabei auf der Mittelachse M der Arthroskopie-Gerade-Schere 1 angeordnet, wobei die Gelenkstäbe 5 jeweils die gleiche Länge aufweisen. Dadurch werden beide Handgriffe 2 gleich weit bei einer Verschiebung des Gleitsteines 4 geöffnet bzw. geschlossen. Je nach Anforderung können diese Verhältnisse aber auch verändert werden.

Am Gleitstein 4 ist weiterhin eine Zugstange 7 befestigt, die in der Verlängerung 6 längsverschieblich entlang der Mittelachse M der Arthroskopie-Gerade-Schere 1 ist. Diese Zugstange 7 kann im Gleitstein 4 beispielsweise mittels eines Gewindes 11 eingedreht und befestigt sein. Durch von herkömmlichen Scheren bekannte Bewegungen der Handgriffe 2 gleitet nun der Gleitstein 4 im Scherenlager 3 durch die Kraftübertragung der Gelenkstäbe 5 auf der Mittelachse M hin und her und überträgt diese Bewegung auf die am Gleitstein 4 befestigte Zugstange 7. Diese weist an ihrem dem Gleitstein abgewandten Ende einen Zapfen 13 auf, an dem weitere Gelenkstäbe 12 befestigt sind. Diese Gelenkstäbe 12 bilden zusammen mit dem scharfen Schneidenteil 14 vom Drehpunkt 10 abgewandten Ende kniehebel 16, die die Hin- und Herbewegung der Zugstange 7 auf die Schneidenteile 14 übertragen und die Bewegung der Schneiden 14 ermöglichen. Auf diese Weise ist es möglich, mittels geeignet gewählter Länge von Verlängerungshülse 6 un d Zugstange die Schneiden 14 der Arthroskopie-Gerade-Schere 1 nahezu jeder beliebigen Operationstiefe anzupassen. Stets können die Handgriffe 2 außerhalb der Operationsöffnung verbleiben.

Wie aus der Fig. 1 weiterhin ersichtlich ist, erfolgt die Bewegung der Handgriffe 2 und der Schneiden 14 jeweils spiegelsymmetrisch zu ihrer gemeinsamen Mittelachse M. Dadurch wird dem Operateur eine besonders einfache und gute Kontrolle der Arthroskopie-Gerade-Schere 1 des erfindungsgemäßen Besteckes ermöglicht.

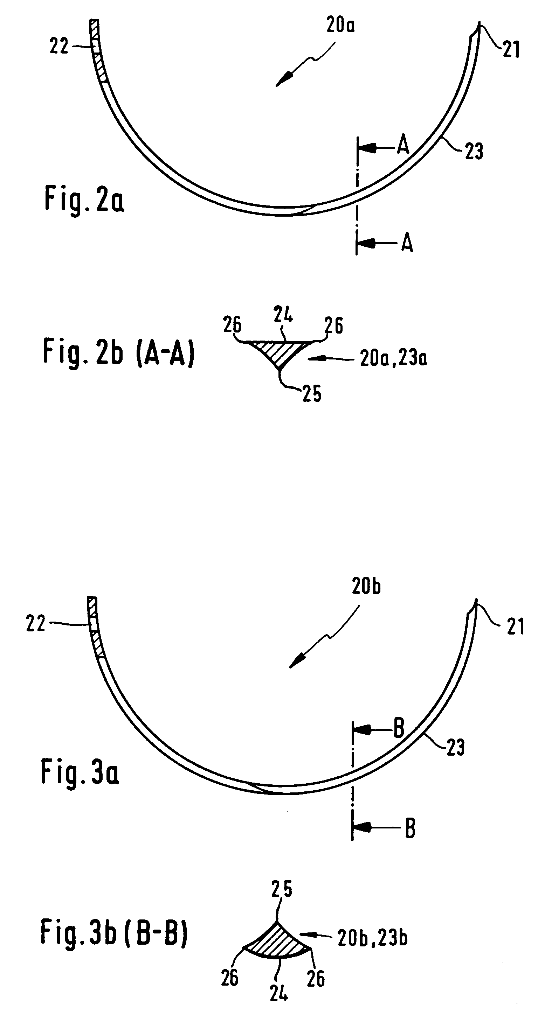

Die Fig. 2a und b eine Sehnenadel 20a des erfindungsgemäßen Besteckes. Die Sehnennadel 20a ist dabei halbkreisförmig abgebogen und weist an ihrem einen Ende eine Spitze 21 und an ihrem anderen Ende eine Öse 22 zum Durchführen eines Operationsfadens auf. Während sie an ihrem Ende, an dem die Öse 22 angebracht ist, einen vorzugsweise runden oder zumindest abgerundeten Querschnitt aufweist, weist die Sehnennadel 20a auf mindestens der Hälfte ihrer Länge, ausgehend von der Nadelspitze 21 einen etwa dreieckigen Keilguerschnitt 23a auf, wie er auch der Fig. 2b zu entnehmen ist. Der keilförmige Querschnitt 23a hat dabei eine dreieckige Form, die von scharfgeschliffenen Ecken 25, 26 und plangeschliffenen Seitenflächen 24 begrenzt wird. Wie die Fig. 2b außerdem zeigt, ist die Keilspitze 25 des keilförmigen Querschnittes 23a der Sehnennnadel 20a am Außenradius der halbkreisförmigen Nadel angeordnet.

Durch diese Ausgestaltung der Sehnennadel mit einem etwa dreieckigen Keilquerschnitt, wobei die Keilspitze am Außenradius der halbkreisförmig gebogenen Nadel angeordnet ist, ermöglicht ein wesentlich verbessertes leichteres Eindringen der Sehnennadel in die Sehne und ruft nur geringere Verletzungen an der Einstichstelle hervor.

Gemäß den Fig. 3a und b weist die Knochennadel des erfindungsgemäßen Besteckes ähnlich zur Sehnennadel, wie sie in den Fig. 2a und b beschrieben ist, eine ebenfalls halbkreisförmig gebogene Gestalt mit einer Spitze 21 und einem weiteren Ende mit Öse 22 zum Durchführen eines Fadens auf. Wiederum ist der Bereich, in dem die Öse 22 angeordnet ist, vorzugsweise von einem runden Querschnitt, mindestens aber einem abgerundeten Querschnitt gebildet, und ein in etwa dreieckiger Keilquerschnitt 23b erstreckt sich auf mindestens der Hälfte der Länge der Knochennadel 20b, ausgehend von der Spitze 21. Im Gegensatz zur oben beschriebenen Sehnennadel 20a weist jedoch die Knochennadel 20b des erfindungsgemäßen Besteckes der Fig. 3b einen in etwa dreieckigen Keilquerschnitt 23b auf, dessen Keilspitze 25 am Innenradius der halbkreisförmig gebogenen Nadel 20b angeordnet ist. Diese Keilspitze 25 begrenzt mit weiteren scharfgeschliffenen Ecken 26 und plangeschliffenen Seiten 24 den etwa dreieckigen Keilquerschnitt 23b.

Eine derartig ausgestattete Knochennadel 20b mit einem keilförmigen Querschnitt 23b, mindestens auf der Hälfte ihrer Länge, ausgehend von der Spitze 21, wobei die Keilspitze 25 am Innenradius der halbkreisförmig gebogenen Nadel 20b angeordnet ist, läßt sich wesentlich besser durch Knochen hindurchschieben und läßt sich leichter beim Durchtritt durch den Knochen führen.

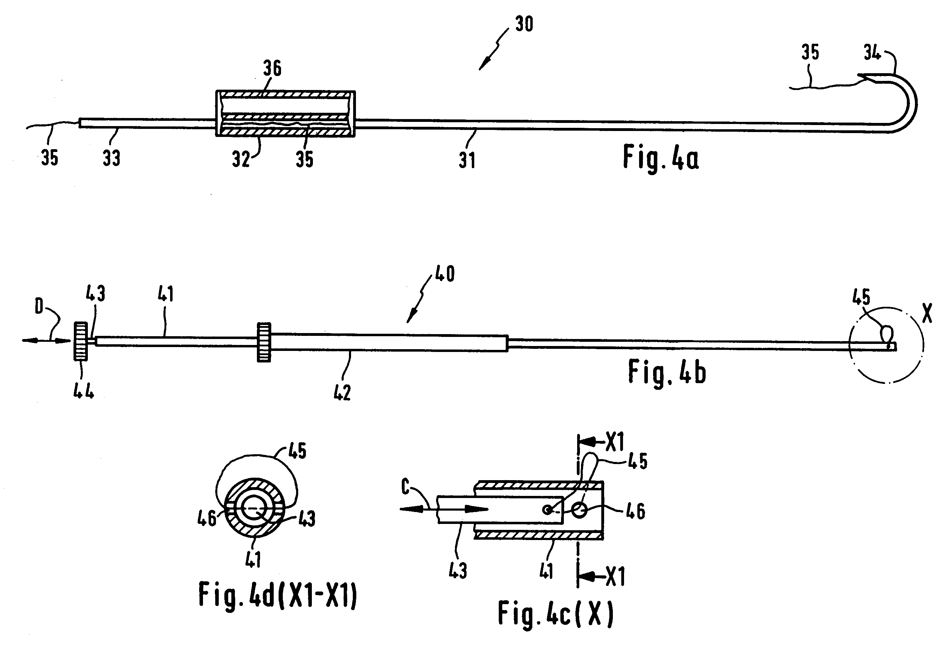

Der Fadenleger 30 des erfindungsgemäßen Besteckes besteht gemäß Fig. 4a aus einer im wesentlichen geraden, durchgehenden Hohlnadel 31, die an ihrem einen Ende 34 hakenförmig abgebogen ist und mit einer scharfgeschliffenen Spitze versehen ist. Ein Operationsfaden 35 kann vom geraden Ende 33 der Nadel 31 hindurch bis an die hakenförmig abgebogene Spitze 34 geschoben werden, aus der er dann austritt. Weiterhin ist eine Führungshülse 32 im hinteren Bereich der überwiegend geraden Nadel 31 des Fadenlegers 30 befestigt, die eine Durchtrittsbohrung 36 zum Durchschieben des Fadenfängers aufweist. Die Durchtrittsbohrung 36 ist dabei derart ausgerichtet, daß sie eine im wesentlichen parallele Führung des Fadenfängers zum geraden Teil 31 des Fadenlegers 30 ermöglicht.

Der Fadenfänger 40 des erfindungsgemäßen Besteckes gemäß Fig. 4b enthält einen äußeren Hohlzylinder 41 mit darauf aufgebrachter Verdickung 42 zum Durchführen durch die Durchführungsbohrung 36 des Fadenlegers 30 und einen inneren Zylinder 43, der im Hohlzylinder 41 längsverschiebbar ist. Gemäß der Fig. 4c und 4d ist eine Drahtschlaufe 45 am Ende des inneren Zylinders 43 befestigt und durch Durchführungsöffnungen 46 nach außen geführt und als Fangschlaufe für einen Operationsfaden. Durch Bewegung des am Ende des inneren Zylinders 43 angebrachten Knaufs 44 in Pfeilrichtung D bewegt sich gemäß Fig. 4c der innere Zylinder 43 gemäß dem Pfeil C längs des äußeren Zylinders 41. Dabei wird die Drahtschlaufe 45 entweder ins Innere des äußeren Zylinders 41 hineingezogen und legt sich an dessen Oberfläche an oder wird aus dem Inneren des äußeren Zylinders 41 herausgeschoben und bildet eine Fangschlaufe.

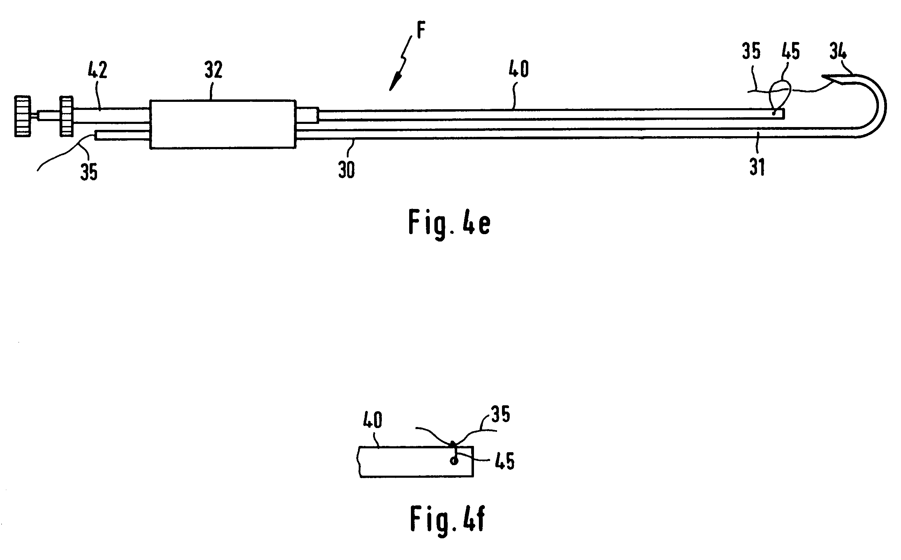

Wenn nun gemäß der Fig. 4e der Fadenleger 30 und der Fadenfänger 40 zu einem kombinierten Fadenleger und -fänger F des erfindungsgemäßen Besteckes zusammengeschoben werden, was durch Einführen der Verdickung 42 in die Durchführungsöffnung 36 des Führungszylinders 32 erfolgt, kann ein aus dem abgebogenen Ende 34 des Fadenlegers austretender Operationsfaden 35 durch die weit offenstehende Drahtschlaufe 45 des Fadenfängers 40 geführt werden. Nach dem bereits beschriebenen Zuziehen der Drahtschlaufe, wie es die Fig. 4b und 4c zeigt, wird der Faden gemäß der Fig. 4f von der eng am Fadenfänger 40 anliegenden Drahtschlaufe 45 festgehalten und kann beispielsweise mitsamt dem Fadenfänger 40 weitergezogen werden.

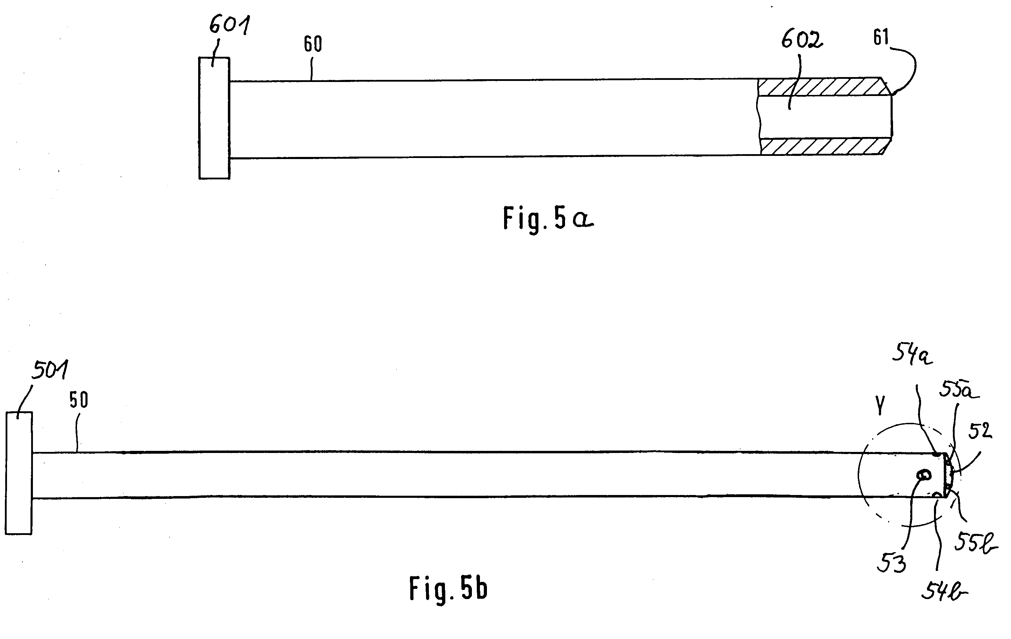

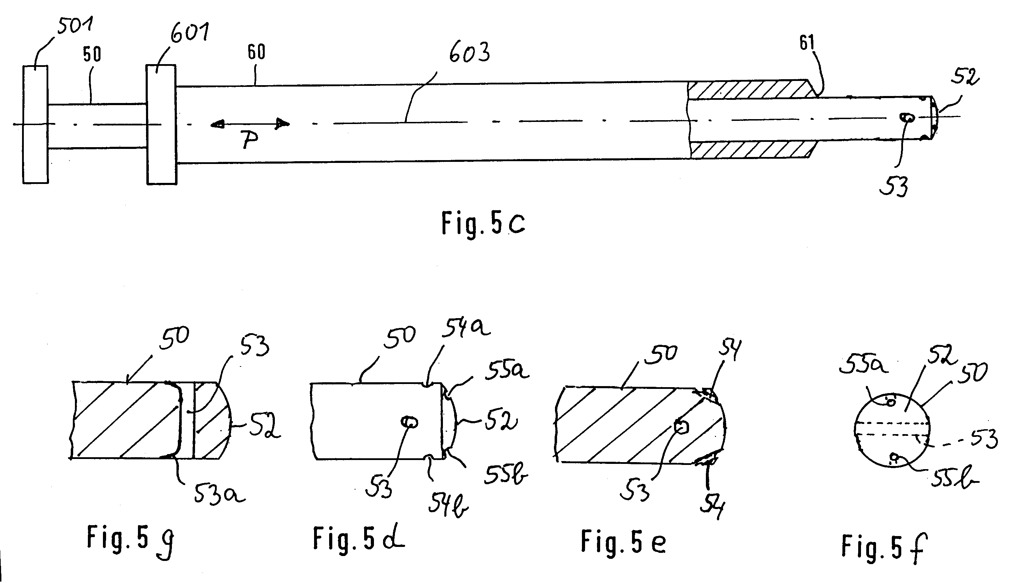

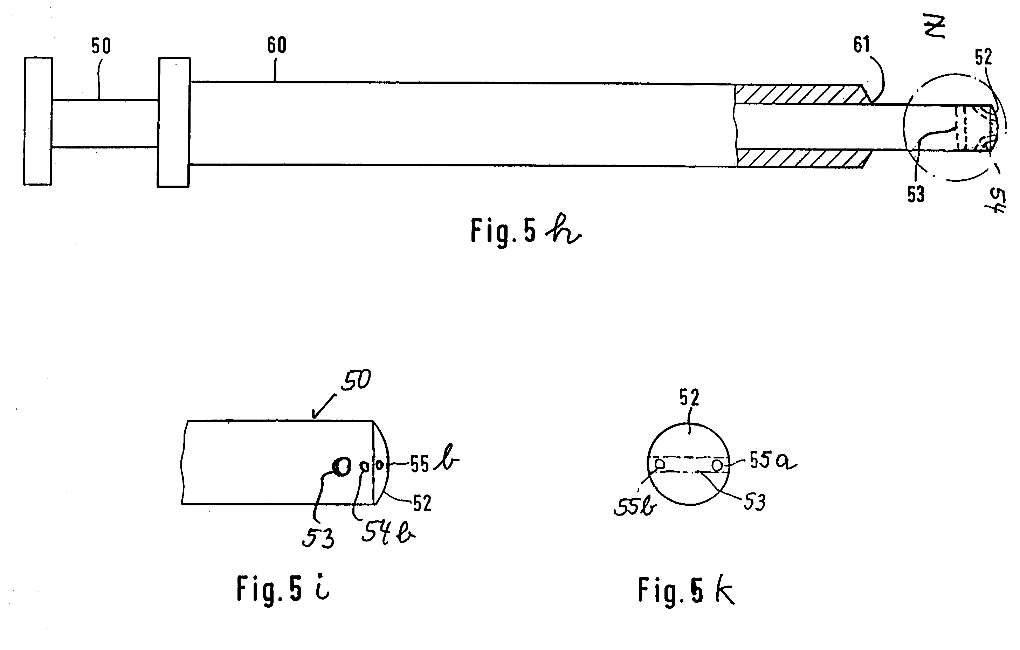

Der Knotenschieber und Knotencutter gemäß der Erfindung, wie er in der Fig. 5c dargestellt ist, setzt sich aus einem zylindrischen Stab 50, siehe Fig. 5b und einer zylindrischen Hülse 60, siehe Fig. 5a, zusammen. Der zylindrische Stab 50 gemäß Fig. 5b weist an einem Ende ein Griffende 501 in Gestalt eines Flanschteiles auf und am anderen Ende ein abgerundetes Kopfende 52 mit verschiedenen Bohrungen zum Durchziehen von Fäden zur Ausübung verschiedener Funktionen. Die zylindrische Hülse, siehe Fig. 5a, weist eine durchgängige Bohrung 602 auf, durch die der zylindrische Stab 50 hindurchsteckbar und längsverschieblich in der Hülse 60 ist. An dem einen Ende weist die Hülse 60 ebenfalls ein Griffende mit vorstehendem Flanschteil 601 auf, an dem der in die Hülse 60 eingesteckte Stab 60 anschlagen kann, siehe Fig. 5c, wenn er in der Längsachse 603 in Pfeilrichtung P bewegt wird. Am anderen Ende weist die Hülse 60, siehe Fig. 5a, eine an die durchgehende Bohrung 602 angrenzende umlaufende Schneidkante 61 auf. Das Detail Y, d.h. das Kopfende des zylindrischen Stabes 50 gemäß Fig. 5b ist in den vergrößerten Detaildarstellungen gemäß Fig. 5d-g dargestellt. Das Kopfende 52 des Stabes 50 ist abgerundet, konvex ausgebildet. Von diesem stirnseitigen Kopfende 52 gehen zwei kleine Bohrungen schräg nach außen zur zylindrischen Mantelfläche des Stabes 50 als durchgehende Bohrung zum Durchziehen eines Fadens. Diese durchgehenden Bohrungen 54, die praktisch ein Nadelöhr darstellen, haben auf der Stirnseite die Eintrittsöffnungen 55a bzw. 55b und an der Mantelfläche die Austrittsöffnungen 54a, 54b. Diese Bohrungen 54, die vorzugsweise symmetrisch am Kopfende einander gegenüberliegend angeordnet sind, sind relativ kurz und enden nicht weit vom stirnseitigen Kopfende des Stabes. Um ein besonders einfaches Hindurchschieben eines Fadens durch diese Bohrungen 54 zu ermöglichen, sind diese schräg in bezug auf die Längsachse des Stabes und zur Mantellinie des zylindrischen Stabes 50 ausgeführt. Des weiteren ist im Bereich des Kopfendes des Stabes 50 nahe den Austrittsöffnungen 54a,b eine weitere Durchgangsbohrung in dem Stab angeordnet, und zwar quer zur Längserstreckung des Stabes, insbesondere mittig. Diese querverlaufende Bohrung 53, die im Querschnitt in Fig. 5g ersichtlich ist, ist in ihren Eintrittsbereichen an der Mantelfläche des zylindrischen Stabes 50 vorzugsweise abgerundet ausgebildet, zumindest in den dem Kopfende 52 abgewandten Bereichen, siehe Abrundungen 53a.

In der Fig. 5h ist eine weitere Variante des Knotenschiebers und Knotencutters dargestellt, bei dem lediglich die Anordnung der querverlaufenden Durchgangsbohrung 53 variiert im Vergleich zu dem zylindrischen Stab des Knotenschiebers und Knotencutters gemäß Fig. 5c. Das in den Fig. 5i und 5k dargestellte Detail Z des zylindrischen Stabes 50 gemäß Fig. 5h in Vergrößerung zeigt, daß die Durchgangsbohrung im Vergleich zu der Ausführung gemäß Fig. 5c um 90° versetzt verläuft.

Mit dem erfindungsgemäß ausgebildeten Knotenschieber und Knotencutter ist es möglich, einen Faden, welcher beispielsweise vom bereits beschriebenen Fadenleger und - fänger gelegt worden ist, durch die Durchgangsbohrungen 54 zu führen und vor der konvex abgerundeten Stirnseite 52 des zylindrischen Stabes 50 zu verknoten. Beim Einführen in die Operationsöffnung des Patienten wird dann der vor der Stirnseite des zylindrischen Stabes liegende Knoten vor dem zylindrischen Stab hergeschoben und kann so in den in einer gewissen Tiefe liegenden Operationsraum gebracht werden. Zum Straffen des Knotens ist es lediglich erforderlich, ein Ende des Knotens durch die durchgehende Bohrung 53 zu führen und an beiden Enden des Knotens Zug auszuüben, wodurch sich der Knoten verfestigt. Selbstverständlich ist es ebenfalls möglich, das eine Knotenende durch beide Bohrungen 54 hindurchzuführen und die Knotenverfestigung vorzunehmen.

Um einen derartig verfestigten Knoten dicht an der Verknotungsstelle vom übrigen Faden zu trennen, weist die auf dem zylindrischen Stab längsverschiebbare zylindrische Hülse 60 an ihrem dem stirnseitigen Ende 52 des zylindrischen Stabes 50 zugewandten Ende eine scharfe Schneidkante 61 auf. Die Knotenenden, welche durch die durchgehenden Bohrungen 54 im zylindrischen Stab 50 von den Öffnungen 55 ausgeführt sind, werden so beim Vorschieben der Hülse 60 von dem scharfen Anschliff 61 am Umfang des zylindrischen Stabes 50 durchtrennt.

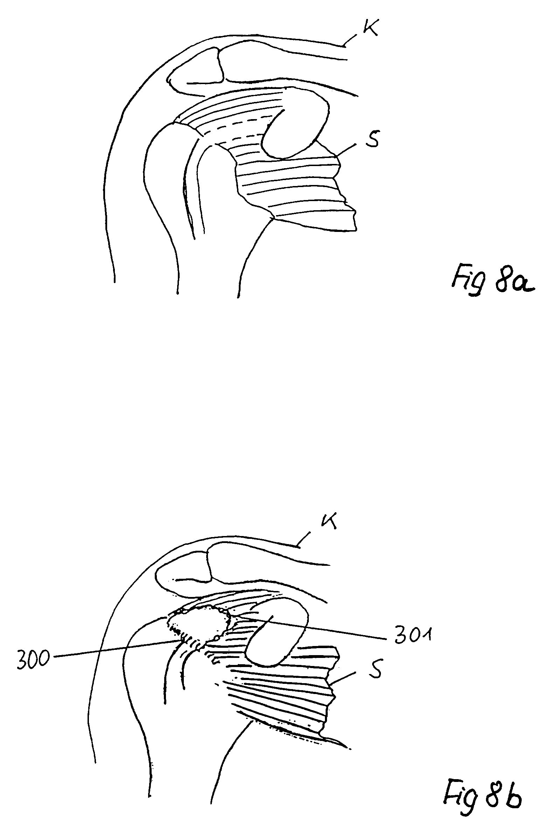

Mit dem erfindgungsgemäßen Besteck läßt sich eine operative Rekonstruktion eines Rotaren-Manschettenrisses mittels eines für den Patienten im Gegensatz zur konventionellen Methode wesentlich angenehmeren transarthroskopischen Eingriffes vornehmen, was nachfolgend anhand der Figuren 8a-h erläutert wird.

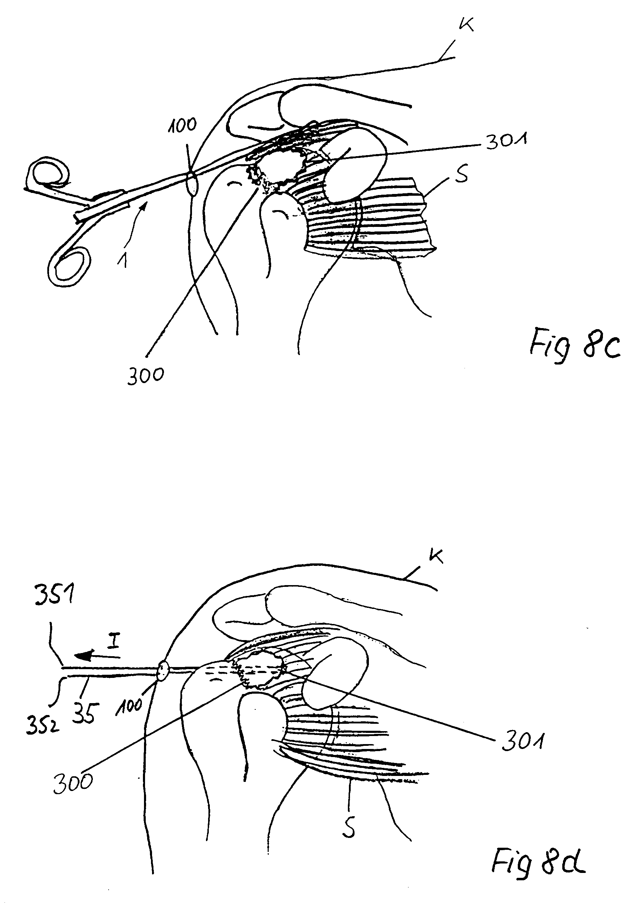

In der Fig. 8a ist der Schulterbereich eines gesunden Patienten schematisch durch Umrißlinien K dargestellt, wobei die Sehnen S unverletzt an den Knochen befestigt sind. Im Gegensatz dazu zeigt die Fig. 8b einen Rotatoren-Manschettenriß dergestalt, daß die Sehne S entlang einer Linie 301 von ihrer ursprünglichen Befestigungsposition 300 am Knochen abgerissen ist. Gemäß der Fig. 8c wird zur Vorbereitung des transarthroskopischen Eingriffes eine Öffnung 100 an geeigneter Stelle am Körper K angebracht, durch den die Elemente des erfindungsgemäßen Besteckes einführbar sind. In einem ersten Schritt werden eventuell vorhandene Verklebungen der Sehne an Knochen mit der Arthroskopie-Gerade-Schere 1 des erfindungsgemäßen Besteckes beseitigt. Zur Unterstützung dieser Arbeit wird auch die Sehnenraspel des erfindungsgemäßen Besteckes je nach Schwere der Verklebungen verwendet. Bei der Sehnenraspel handelt es sich um eine kleine Metallraspel, die besonders klein ausgebildet ist, so daß sie durch die Öffnung 100 ein- und ausführbar ist.Die ursprüngliche Stelle 300, an der die Sehne S entlang ihrer Linie 301 am Knochen befestigt war, wird durch eine entsprechende Vorbehandlung mittels Fräser und Raspel auf die Rekonstruktion vorbereitet, was im wesentlichen bedeutet, daß eine glatte Oberfläche und eine Rille im Knochen eingearbeitet wird.

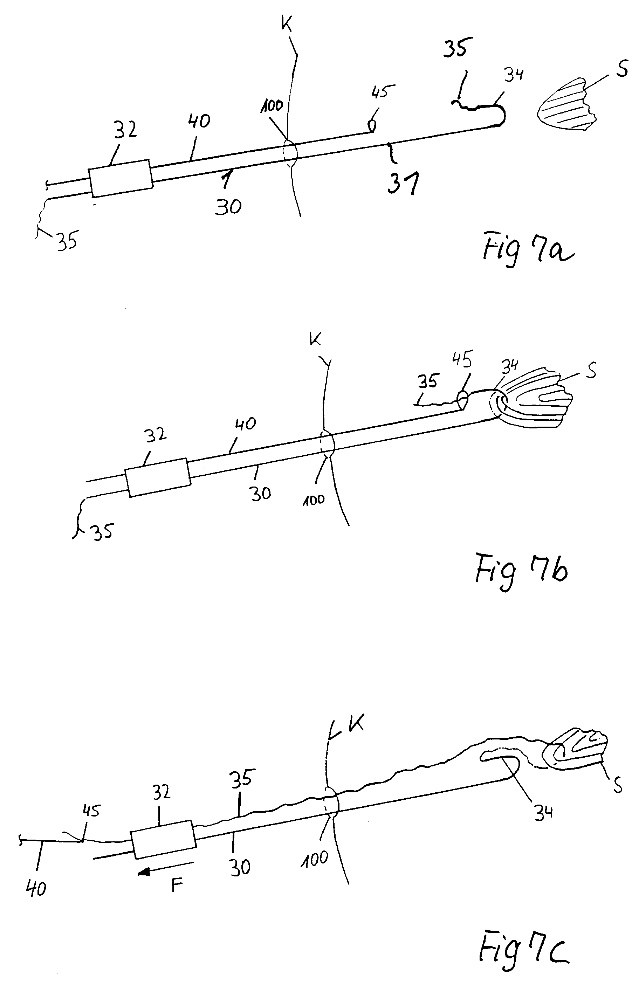

In einem nächsten Schritt wird mit dem Fadenleger und -fänger die Sehne im Bereich 301 durchstochen und ein Faden gelegt, wie es beispielsweise in den Fig. 7a-c dargestellt ist. Hierbei zeigt Fig. 7a das Einschieben des hakenförmigen Endes 34 der Hohlnadel 31 des Fadenlegers 30 mit dem Faden 35, der durch die Hohlnadel 31 gezogen ist durch die Körperöffnung und durch die Sehne S, siehe Fig. 7b. Dann wird der Fadenfänger 40, siehe Fig. 7b, nachgeschoben, um das Ende des Fadens 35 einzufangen und festzuhalten mit Hilfe der Drahtschlaufe 45. Nunmehr wird das eingefangene Ende des Fadens 35 mit dem Fadenfänger 40 in Pfeilrichtung F, siehe Fig. 7c, aus dem Körper herausgezogen. Auch der Fadenleger 30 wird aus dem Körper herausgezogen, so daß die beiden Fadenenden 351, 352 des Fadens 35 außerhalb des Körpers sind und der Faden durch die Sehnen geführt ist, siehe Fig. 8d. Es werden mit dem Fadenleger und -fänger zwei bis drei Fäden gelegt, die zum Halten der abgerissenen Sehne in der Position zum Vernähen dienen. Mit diesen Fäden 35 wird die abgerissene Sehne auch in die gewünschte Position gezogen. Die Fig. 8d zeigt die erfolgreiche Legung eines derartigen Fadens 35 durch die Operationsöffnung 100 im Köper K. Durch Zug in Pfeilrichtung I kann nun die Sehne über den Knochen auf ihren ursprünglichen Platz 300 gezogen werden.

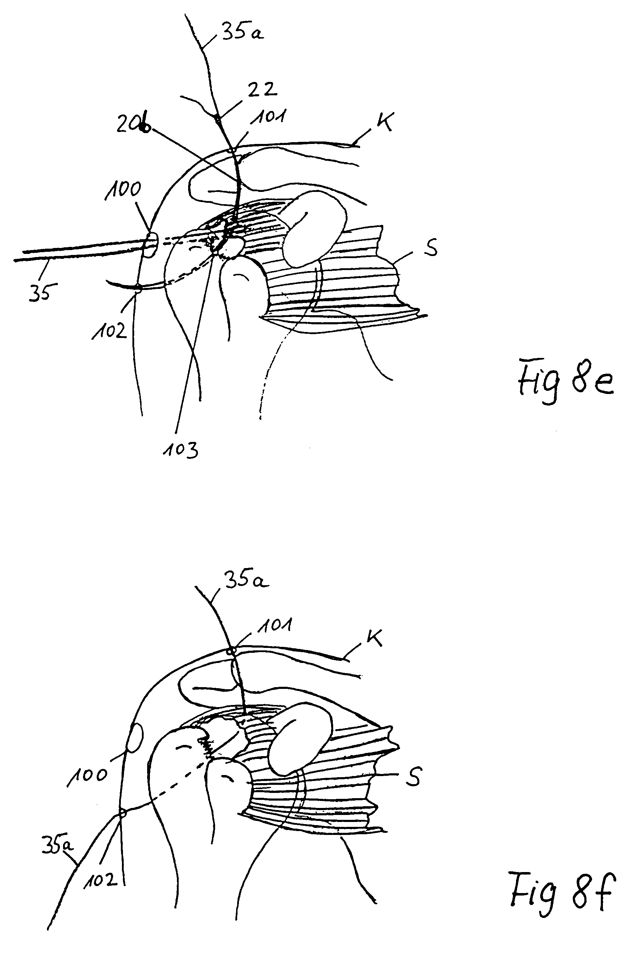

Im nächsten Schritt wird die derartig in ihrer ursprünglichen Lage mittels der gelegten Fäden 35 gehaltene Sehne gemäß der Fig. 8e von einer Knochennadel 20b, welche durch eine weitere Öffnung 101 im Körper K eingeführt ist, durchstochen und die Knochennadel wird durch eine Öffnung 103 im Knochen und eine weitere Öffnung 102 wieder aus dem Körper K herausgeführt. Dabei zieht sie einen Faden 35a, der durch die Öse 22 der Knochennadel 20b gezogen ist, entlang ihres Weges durch den Körper hinter sich her, so daß der Faden 35a gemäß der Fig. 8f zur Ausbildung einer transossären Sehnennaht zum Liegen kommt. Sodann kann der Unterstützungsfaden 35 der zuvor gemäß Fig.8d gelegt worden ist, wieder entfernt werden, so daß die Sehne S wiederum zurückschnellt. Sollte die Sehne längs gerissen sein, kann man mit einer langen speziellen Nadel, siehe Fig. 2a,b, die eine konkave Verflachung aufweist, einen Faden 35a durch die Ränder des Risses von vorne nach hinten als sogenannte End-zu-End-Naht durchführen.

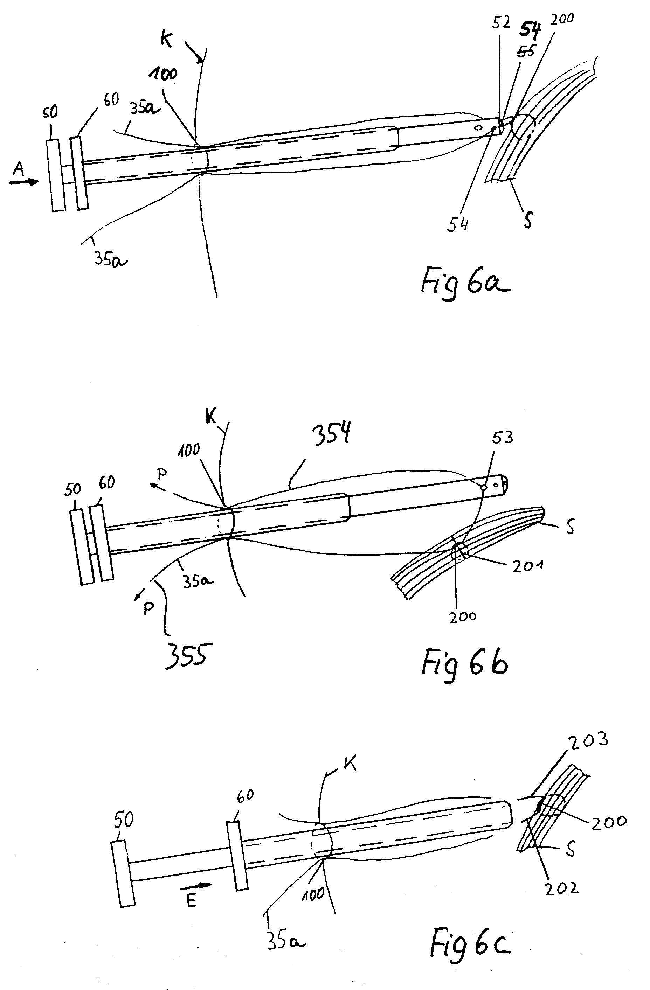

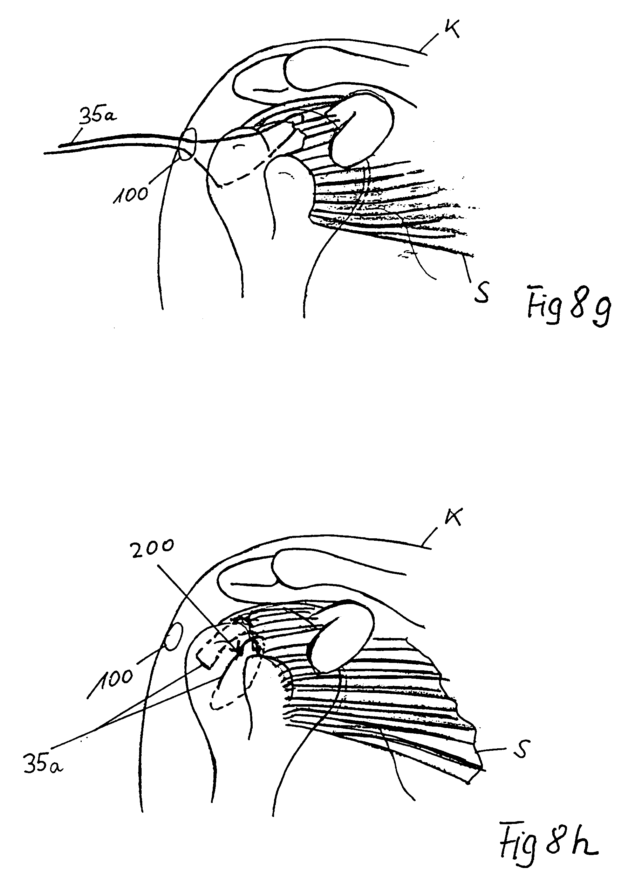

Gemäß der Fig. 8g wird der von der Knochennadel gelegte Faden 35a mit einem entsprechenden Haken aus der Operationsöffnung 100 des Körpers K herausgeführt und kann mit dem Knotenschieber und -cutter 50, 60 gemäß Fig. 5a bis k, wie in den Fig. 6a-c dargestellt, verknotet werden, so daß die Sehne S gemäß der Fig. 8h wieder auf ihrem ursprünglichen Platz am Knochen unter Belassung eines Knotens 200 im Faden 35a fixiert wird. Hierbei wird zuerst ein Knoten 200 mit den Fadenenden des Fadens 35 außerhalb des Körpers K geknotet. Dann wird der Faden 35a durch die beiden Bohrungen 54 am Kopfende des Knotenschiebers 50, siehe auch Fig. 5a bis 5k gelegt, und dann wird der außerhalb des Körpers geknotete Knoten in den Körper bis zum Sehnenende hin mit dem Knotenschieber geschoben, siehe Fig. 6a. Dann wird der Knotenschieber und -cutter wieder herausgezogen und ein Ende 354 des Fadens 35a durch die Querbohrung 53 des Knotenschiebers gelegt, das andere Fadenende 355 wird festgehalten, siehe Fig. 6b, der Knotenschieber wird dann wieder eingeführt und der Knoten 200 wird im Gegenzug durch Ziehen an den Fadenenden 354, 355 gefestigt.

Nach dem ersten Knoten wird ein weiterer Knoten gelegt, so daß der erste Knoten fest ist. Danach wird die Hülse 60 in Pfeilrichtung E, siehe Fig. 6c, vorgeschoben bis über den Knoten und dabei die überstehenden Fadenenden des Fadens 35a mit der Schneidkante 61, siehe auch Fig. 5a, abgeschnitten. Damit ist die operative transarthroskopische Rekonstruktion des Rotatoren-Manschettenrisses beendet. Mit Hilfe des Knotenschiebers und -cutters wird so eine Längsnaht und eine O-Naht gelegt und dabei die Sehne am Knochen wieder in einer anatomischen Stellung befestigt.

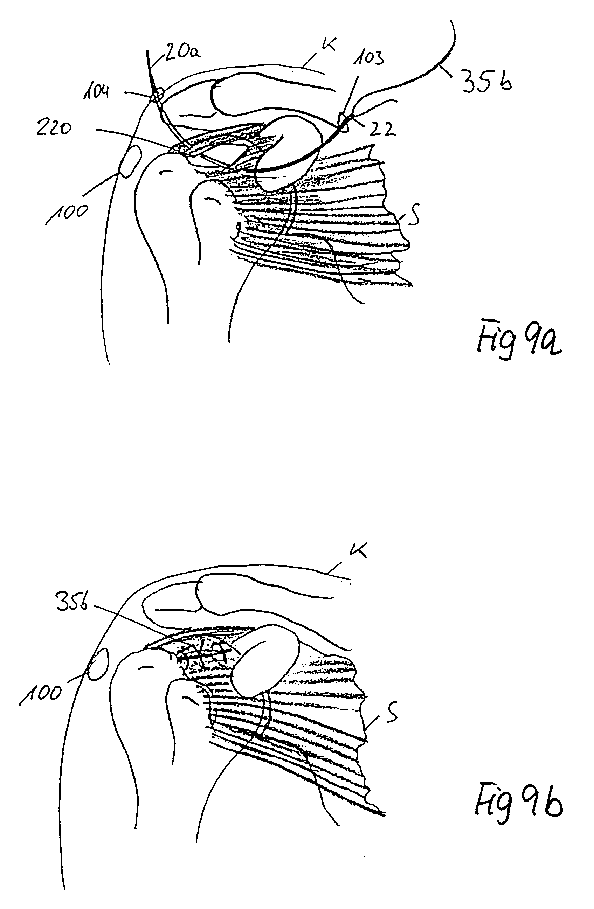

Die Fig. 9a und b zeigen einen weiteren möglichen Rotatoren-Manschettenriß, bei dem die Sehne S gemäß Fig. 9a einen Längsriß 220 aufweist. Nach dem Legen der Fäden mit dem Fadenleger und -fänger wird eine Nadel 20a gemäß Fig. 2a,b in diesem Fall durch die Öffnung 103 in den Körper K eingeführt und beiderseits des Längsrisses 220 die Sehne S durchstochen. Die Nadel 20a verläßt den Körper durch die Öffnung 104 wieder und zieht dabei längs ihres Weges den Faden 35b, der in der Öse 22 der Nadel 20a befestigt ist, hinter sich her. Nun kann in bereits dargestellter Weise mit dem Knotenschieber und - cutter durch die Operationsöffnung 100 der außerhalb hergestellte Knoten des Fadens mittels des Knotenschiebers an die gewünschte Position verschoben werden, danach wie bei Fig. 6a-c beschrieben, festgezogen werden, wobei dieser Vorgang mehrfach wiederholt wird und zum Schluß die überstehenden Fadenenden mit dem Cutter abgetrennt werden. Damit ist die Sehne S wieder in ihre ursprüngliche Lage gebracht.

Damit ist auch dieser operative transarthroskopische Eingriff zur Rekonstruktion des Rotatoren-Manschettenrisses abgeschlossen.

Die erfindungsgemäßen Bestecke bieten also für alle möglichen Varianten eines möglichen Rotatoren-Manschettenrisses eine einfache Möglichkeit, den Eingriff zur Rekonstruktion auf patientenschonende transarthroskopische Weise durchzuführen, wobei das erfindungsgemäße Besteck auch für andere operative transarthroskopische Eingriffe verwendbar ist.

## Patentansprüche

1. Medizinisches Besteck zum Verschieben und Plazieren von mittels chirurgischer Fäden hergestellter Knoten bei operativen Eingriffen mit einem in einer zylindrischen Hülse (60) längsverschieblichen zylindrischen Stab (50), der an seinen beiden Enden über die Hülse (60) vorsteht, wobei der Stab (50) ein in die Hülse (60) hineinziehbares Kopfende (52) und ein am anderen Ende (601) der Hülse (60) außenseitig anschlagbares Griffende (501) aufweist und von dem stirnseitigen Kopfende (52) sich zwei durchgehende Bohrungen (54) zum Durchstecken eines Fadens zur zylindrischen Mantelfläche des Stabes erstrecken und das Hülsenende, in das der Stab (50) einziehbar ist, als Knotencutter mit Schneidkante (61) ausgebildet ist, um die Fadenenden vom Knoten abzutrennen, **dadurch gekennzeichnet**, daß eine weitere Durchgangsbohrung (53) zum Durchziehen eines Fadens im Bereich des Kopfendes des Stabes (50) ausgebildet ist, die quer zur Längsachse (603) des zylindrischen Stabes angeordnet ist, wodurch das Kopfende (52) des zylindrischen Stabes als Knotenschieber zum Verschieben und Festziehen eines mittels der Fäden hergestellten Knotens ausgebildet ist.

2. Besteck nach Anspruch 1,
**dadurch gekennzeichnet**, daß der zylindrische Stab (50) als Knotenschieber mit einem konvex abgerundeten Kopfende (52) ausgebildet ist.

3. Besteck nach Anspruch 1 oder 2,
**dadurch gekennzeichnet**, daß die beiden durchgehenden vom abgerundeten Kopfende ausgehenden Bohrungen (54) diametral einander gegenüberliegend am Kopfende des Stabes in bezug auf die Längsachse des Stabes (50) ausgebildet sind, wobei die Bohrungen von der Stirnseite des Kopfendes schräg nach außen zur zylindrischen Mantelfläche des Stabes hin verlaufend angeordnet sind.

4. Besteck nach einem der Ansprüche 1 bis 3,
**dadurch gekennzeichnet**, daß die Bohrungen (54) 2 bis 4 mm lang sind und einen Durchmesser von 1 bis 2 mm aufweisen.

5. Besteck nach Anspruch 1,
**dadurch gekennzeichnet**, daß die querverlaufende Durchgangsbohrung (53) im Eintrittsbereich bzw. Austrittsbereich an der Mantelfläche des zylindrischen Stabes zumindest in dem dem Kopfende (52) abgewandten Bereichen abgerundet (53a) ausgebildet ist.

6. Besteck nach einem der Ansprüche 1 oder 5,
**dadurch gekennzeichnet**, daß die querverlaufende Durchgangsbohrung (53) einen größeren Durchmesser als die Bohrung (54) aufweist.

## Claims

1. Set of medical instruments for shifting and placing knots produced by surgical sutures in surgery with a cylindrical rod (50) displaceable lengthwise and a cylindrical sleeve (60), said rod projecting at its two ends beyond sleeve (60), with rod (50) having one head end (52) that can be retracted into sleeve (60) and a grip end (501) strikable externally at the other end (601) of sleeve (60), and with two through bores (54) extending from head end (52) for passing a suture to the cylindrical jacket surface of the rod, and with the sleeve end into which rod (50) can be retracted being designed as a knot cutter with a cutting edge (61) to separate the suture ends from the knots,
characterized in that an additional through bore (53) is designed for pulling a suture through in the vicinity of the head end of rod (50), said rod being located transversely with respect to lengthwise axis (603) of the cylindrical rod, with head end (52) of the cylindrical rod being designed as a knot shifter for shifting and tightening a knot produced by means of the sutures.

2. Set of instruments according to Claim 1, characterized in that cylindrical rod (50) is designed as a knot shifter with a convexly rounded head end (52).

3. Set of instruments according to Claim 1 or 2, characterized in that the two through bores (54) running from the rounded head end are formed diametrally opposite one another at the head end of the rod, relative to the lengthwise axis of rod (50), with the bores running from the end of the head end diagonally outward to the cylindrical jacket surface of the rod.

4. Instruments according to one of Claims 1 to 3, characterized in that bores (54) are 2 to 4 mm long and have a diameter of 1 to 2 mm.

5. Instruments according to Claim 1, characterized in that through bore (53) running crosswise in the entrance area or outlet area on the jacket surface of the cylindrical rod is made rounded (53a) at least in the areas that face away from head end (52).

6. Instruments according to one of Claims 1 or 5, characterized in that through bore (53) running crosswise has a diameter larger than bore (54).

## Revendications

1. Instrument médical pour déplacer et placer des noeuds réalisés avec du fil chirurgical lors d'interventions chirurgicales, comportant une tige cylindrique (50) qui est montée coulissante longitudinalement dans un manchon cylindrique (60) et dépasse par rapport au manchon (60) à ses deux extrémités, la tige (50) présentant une extrémité tête (52) qui peut être rentrée dans le manchon (60) et, à l'autre extrémité (601) du manchon (60), une extrémité poignée (501) qui peut être saisie extérieurement, deux trous traversants (54) pour le passage d'un fil s'étendant depuis la face frontale de l'extrémité tête (52) en direction de la surface extérieure cylindrique de la tige, et l'extrémité du manchon dans laquelle la tige (50) peut être rentrée étant agencée en moyen de coupe de noeud pourvu d'un bord coupant (61) pour séparer les extrémités du fil du noeud, caractérisé par le fait qu'un trou traversant (53) supplémentaire pour le tirage d'un fil est aménagé dans la région de l'extrémité tête de la tige (50), lequel trou est disposé transversalement à l'axe longitudinal (603) de la tige cylindrique, l'extrémité tête (52) de la tige cylindrique étant ainsi agencée en un moyen de déplacmeent et de serrage d'un noeud réalisé à l'aide de fils.

2. Instrument selon la revendication 1, caractérisé par le fait que la tige cylindrique (50) en tant que moyen pour déplacer le noeud est pourvue d'une extrémité tête (52) arrondie convexe.

3. Instrument selon la revendication 1 ou 2, caractérisé par le fait que les deux trous traversants (54) qui s'étendent à partir de l'extrémité arrondie de l'extrémité tête sont diamétralement opposés l'un a l'autre à l'extrémité tête de la tige par rapport à l'axe longitudinal de la tige (50), les trous étant inclinés vers l'extérieur depuis la face frontale de l'extrémité tête en direction de la surface extérieure de la tige.

4. Instrument selon une des revendications 1 à 3, caractérisé par le fait que les trous traversants (54) ont une longueur comprise dans une plage allant de 2 à 4 mm et un diamètre compris dans une plage allant de 1 à 2 mm.

5. Instrument selon la revendication 1, caractérisé par le fait que le trou traversant (53) transversal dans la zone d'entrée ou dans la zone de sortie dans la surface extérieure de la tige cylindrique présente un arrondi (53a), au moins dans la zone éloignée de l'extrémité de tête (52).

6. Instrument selon une des revendications 1 ou 5, caractérisé par le fait que le trou traversant (53) transversal présente un diamètre supérieur au trou (54).
